# FASCICULE DE BREVET EUROPEEN

(11) **EP 1 523 270 B1**
(45) Date de publication et mention de la délivrance du brevet: **19.05.2010**
(21) Numéro de dépôt: 03755595.0
(22) Date de dépôt: 11.07.2003
(51) Int. Cl.: A61B 5/00, G02B 21/00, G02B 23/24, G02B 23/26

(54) **PROCEDE ET APPAREILLAGE D'IMAGERIE DE FLUORESCENCE HAUTE RESOLUTION PAR FIBRE OPTIQUE ET NOTAMMENT D'IMAGERIE CONFOCALE**
VERFAHREN UND VORRICHTUNG ZUR HOCHAUFLÖSENDEN FLUORESZIERENDEN ABBILDUNG MIT OPTISCHEN FASERN UND INSBESONDERE KONFOKALE ABBILDUNG
METHOD AND EQUIPMENT FOR FIBER OPTIC HIGH-RESOLUTION, IN PARTICULAR CONFOCAL, FLUORESCENCE IMAGING

(30) Priorité: 18.07.2002 FR 0209099; 11.03.2003 FR 0302972
(43) Date de publication de la demande: 20.04.2005
(60) Demande divisionnaire: 08159710.6
(73) Titulaire: Mauna Kea Technologies, 75010 Paris (FR)
(72) Inventeur: GENET, Magalie, F-78280 Guyancourt (FR); VIELLEROBE, Bertrand, F-94130 Nogent sur Marne (FR); BERIER, Frédéric, F-29810 Plouarzel (FR); CLADE, Sophie, F-75016 Paris (FR)
(74) Mandataire: Pontet, Bernard
(86) Numéro de dépôt international: PCT/FR2003/002196
(87) Numéro de publication internationale: WO 2004/008952

(56) Documents cités:
- WO-A-00/16151
- US-A- 3 753 607
- US-A- 5 672 880
- US-A- 5 719 391
- US-A- 5 737 121
- US-A1- 2002 045 811
- US-B1- 6 370 422
- US-B1- 6 400 487
- KNITTEL J ET AL: "Endoscope-compatible confocal microscope using a gradient index-lens system" OPTICS COMMUNICATIONS, NORTH-HOLLAND PUBLISHING CO. AMSTERDAM, NL, vol. 188, no. 5-6, 15 février 2001 (2001-02-15), pages 267-273, XP004317426 ISSN: 0030-4018 cité dans la demande
- ROUSE A R ET AL: "MULTISPECTRAL IMAGING WITH A CONFOCAL MICROENDOSCOPE" OPTICS LETTERS, OPTICAL SOCIETY OF AMERICA, WASHINGTON, US, vol. 25, no. 23, 1 décembre 2000 (2000-12-01), pages 1708-1710, XP000981049 ISSN: 0146-9592
- Gmitro A.F. et al.: "Confocal microscopy through a fiber-optic imaging bundle", Optics Letters, Vol. 18, No. 8, 1 pages 565-567
- Rouse A.R. et al.: "Development of a fiber-optic confocal microendoscope for clinical endoscopy"; Proceedings of SPIE, Vol. 4613, pages 244-253, 01.05.02

## Description

La présente invention concerne un procédé et un appareillage d'imagerie de fluorescence fibrée haute résolution, notamment confocale. Le domaine d'application visé est plus particulièrement celui de l'imagerie in vivo et in situ.

La fluorescence observée peut provenir d'un composé exogène (typiquement un marqueur injecté) ou endogène (présent dans la cellule) d'un tissu biologique.

Plus particulièrement, le procédé d'imagerie confocale selon l'invention est du type consistant à balayer point par point un tissu dans un plan subsurfacique, chaque point correspondant à un signal d'excitation émis par une source continue, dévié et injecté tour à tour dans une fibre optique d'un faisceau de fibres optiques, puis focalisé à la sortie de ladite fibre dans ledit plan, chaque point émettant en retour un signal de fluorescence collecté par ladite fibre optique, puis détecté et numérisé pour former un élément d'image.

Le caractère confocal est obtenu en utilisant le même chemin optique, notamment la même fibre optique servant de filtrage spatial, pour transporter le signal d'excitation et le signal de fluorescence émis en réaction, et en utilisant un système optique permettant de conjuguer le point de focalisation dans le tissu avec ladite fibre optique.

L'appareillage d'imagerie confocale correspondant comprend le faisceau de fibres optiques souples (appelé couramment guide d'image) avec, à son extrémité proximale :
- la source émettant en continu à la longueur d'onde d'excitation d'un ou plusieurs fluorophores ciblés, typiquement une source laser ;
- des moyens de balayage rapide dans le temps du faisceau d'excitation produit par la source en lignes et en colonnes dans un plan XY correspondant à la section d'entrée du guide d'image ;
- des moyens d'injection du faisceau d'excitation dans l'une des fibres optiques ;
- des moyens de détection du signal de fluorescence ; et
- des moyens de commande, notamment des moyens de balayage.

Des moyens étant en outre prévus pour permettre la réalisation et la visualisation d'une image à partir des signaux détectés successivement sur chaque fibre.

A l'extrémité distale du guide d'image, se trouve une tête optique, destinée à être mise en contact avec le tissu observé, permettant de focaliser le faisceau d'excitation à une profondeur donnée (quelques dizaines de µm) et donc de réaliser une image subsurfacique.

En pratique, ce type d'appareillage présente notamment les avantages suivants :
- du côté distal du guide d'image se trouve uniquement une optique de focalisation, qui est moins fragile et moins coûteuse qu'une tête optique intégrant des moyens de balayage, le remplacement de la tête optique pouvant être envisagé indépendamment des moyens de balayage ; par ailleurs la tête est miniaturisable, ce qui présente un intérêt en endoscopie et également, d'une manière générale, pour augmenter la précision de positionnement ;
- un guide d'image fait de fibres optiques souples, sert de bras d'accès au site à observer, ce qui est important pour une application in situ.

Dans l'état de la technique, on a décrit les procédés et appareillages de fluorescence suivants.

Dans un article paru dans "Applied Optics", Vol.38, No. 34, décembre 1999, pages 7133-7144, est présenté un micro-endoscope confocal mettant en oeuvre un faisceau de fibres optiques souples muni à son extrémité distale d'une tête optique miniature de focalisation ; il est prévu de balayer le faisceau de fibres optiques en lignes, l'appareillage comprenant une fente de détection ainsi qu'un détecteur linéaire CCD de type à transfert de charges. Un appareillage de ce type permet d'obtenir 4 images/s, ce qui est trop lent pour une imagerie in situ tributaire des bougés du sujet et de l'opérateur. Par ailleurs, le fait de balayer en lignes et non point par point dégrade le caractère confocal et conduit à une image moins bien résolue.

Dans un article paru dans "Optics Communications", 188 (2001), pages 267-273, est présenté le couplage d'un microscope confocal de table à balayage laser avec un faisceau de fibres optiques souples muni à son extrémité distale d'une tête optique miniature. Le but recherché est de rendre le microscope compatible avec un endoscope. Le balayage est réalisé fibre à fibre mais le microscope de table utilisé est de conception classique, conçu pour visualiser un échantillon fixe sans soucis du temps d'obtention d'une image. Un temps de pose de 2 secondes est avancé dans cet article, beaucoup trop long pour une imagerie in situ, en temps réel.

Dans l'article "Development of a fiber-optic confocal microendoscope for clinical endoscopy", paru dans Proceedings of SPIE, Vol. 4613, pages 244-253, 01.05.02, est présenté un microscope confocal de fluorescence avec un faisceau de fibres optiques qui est balayé en lignes. Le microscope comprend un détecteur CCD opérant à une fréquence de 3 MHz, et il permet d'obtenir 8 images par seconde, les images comprenant 512 x 512 pixels.

La présente invention a pour but de proposer un procédé et un appareillage d'imagerie de fluorescence fibrée haute résolution, notamment confocale, du type utilisant un guide d'image fait de fibres optiques balayées une à une par un faisceau d'excitation émis en continu, lesdits procédé et appareillage permettant la visualisation en temps réel d'un site in vivo in situ, c'est-à-dire capable de fournir un nombre suffisant d'images point à point par seconde sans être tributaire des bougés du sujet et du praticien pour permettre notamment un examen assez rapide.

La présente invention a également pour objet de proposer un procédé et un appareillage qui, d'une manière générale, optimisent la qualité de chaque image afin notamment d'obtenir une excellente résolution latérale et axiale. Le procédé selon l'invention est défini dans la revendication 1.

La présente invention propose une solution pour réaliser une image in vivo in situ en temps réel. L'invention est basée sur le respect de l'échantillonnage des fibres (selon le critère de Shannon) qui permet d'obtenir et de reconstruire une image point à point correspondant bien à chaque fibre. Cela permet de ne pas perdre d'information en échantillonnant l'ensemble des fibres une à une tout en respectant un nombre moyen minimal d'images par seconde, à savoir en pratique au minimum 12 images par seconde pour un mode maximal de 640 x 640 pixels. Le choix de la fréquence de détection (bande passante du détecteur) en fonction de cet échantillonnage minimal permet ensuite pour chaque fibre de détecter le plus grand nombre possible de photons de fluorescence.

Ainsi, selon un mode de réalisation possible, mettant en oeuvre un guide d'image d'environ 30 000 fibres optiques souples, la fréquence d'échantillonnage et la bande passante du système de détection (une photodiode à avalanche ou équivalent) sont fixés sensiblement à 1,5 MHz, correspondant environ à 12 pixels par fibre, permettant alors d'obtenir au minimum les 12 images/s en mode maximal 640 x 640 pixels.

En pratique, selon un mode de réalisation avantageux, permettant un balayage rapide approprié des fibres pour obtenir une image en temps réel, la déviation du faisceau est réglée en déterminant une fréquence de résonance rapide d'un miroir résonnant « ligne » et une fréquence de résonance lente d'un miroir galvanométrique « trame ».

Selon l'invention, la durée de détection d'un flux en provenance d'une fibre est limitée dans le temps et très courte pour respecter l'acquisition d'une image en temps réel. Selon l'invention, on a donc en outre prévu des moyens d'optimisation pour collecter et détecter le maximum de flux en provenance de l'échantillon pendant cette durée de détection limitée, notamment :
- on utilise des moyens optiques de déviation, d'injection, le cas échéant de focalisation et de détection présentant un degré d'achromaticité dépendant de l'écart en longueur d'onde entre la longueur d'onde d'excitation et la longueur d'onde maximale de fluorescence, ainsi que de la largeur spectrale du signal de fluorescence émis ; cela permet avantageusement d'optimiser le flux de fluorescence collecté sur toute la bande spectrale de fluorescence émise ;
- on choisit, le cas échéant, une ouverture numérique de l'optique de focalisation sur le tissu comprise sensiblement entre 0,5 et 1, permettant d'obtenir un bon compromis entre la taille de champ et la quantité de photons collectée ; et
- on choisit un détecteur ayant une efficacité quantique aux longueurs d'ondes de fluorescence à détecter d'au moins 50%.

Selon l'invention, le traitement d'image réalisé ensuite sur le flux détecté est également optimisé, pour obtenir une image de très bonne qualité à partir de ce flux limité de photons détecté. Cette optimisation est réalisée de la manière suivante.

Une série d'étapes est réalisée préalablement à l'acquisition d'images en temps réel :
- une étape de détection de l'emplacement de chaque fibre d'un ensemble choisi de fibres destinés à être utilisées (soit l'ensemble du guide d'image soit un sous-ensemble choisi) ; cette étape est à réaliser au moins à chaque changement de guide d'image ;
- une étape de calibration du taux d'injection dans chaque fibre, c'est à dire de définition d'un taux d'injection propre à chaque fibre ; et
- une étape de détection de l'image de fond (sans échantillon).

En fonctionnement, l'optimisation du traitement d'image comprend notamment les étapes consistant, après numérisation du signal détecté :
- à définir le flux réel collecté par chaque fibre c'est-à-dire ne provenant que de l'échantillon, après correction en fonction du taux d'injection propre de la fibre et de la soustraction de l'image du fond, de manière à obtenir un signal corrigé;
- puis à effectuer une reconstruction de l'image à partir de ce signal corrigé, avec pour but notamment de transformer une image présentant une mosaïque de fibres en une image sans fibres apparentes.

Selon l'invention, ces deux dernières étapes doivent être réalisables en temps réel. Pour ce qui est de la correction du signal, celle-ci peut se faire en temps réel grâce à un traitement adapté à la structure du signal observé et un algorithme optimisé. Pour ce qui est de la reconstruction de l'image, elle peut se faire grâce au choix d'un nombre d'opérations par pixel réalisables en temps réel permettant d'obtenir le résultat recherché en terme de qualité d'image. Un filtrage passe-bas Gaussien représente un bon compromis entre la complexité du traitement, la qualité du résultat et le temps de calcul.

Pour gagner du temps et augmenter la complexité du traitement correspondant à la reconstruction de l'image, on peut aussi augmenter la capacité de traitement du matériel, par exemple en utilisant une carte de traitement spécifique et/ou une architecture parallèle comme un multiprocesseur.

La présente invention propose également un appareillage tel que défini dans la revendication 13.

La présente invention sera mieux comprise et d'autres avantages apparaîtront à la lumière de la description qui va suivre d'un exemple de réalisation, description faite en référence aux dessins annexés sur lesquels :
- la figure 1 représente schématiquement un exemple de réalisation d'appareillage d'imagerie confocale de fluorescence comprenant une tête de focalisation ;
- la figure 2 est un schéma fonctionnel de l'appareillage de la figure 1.

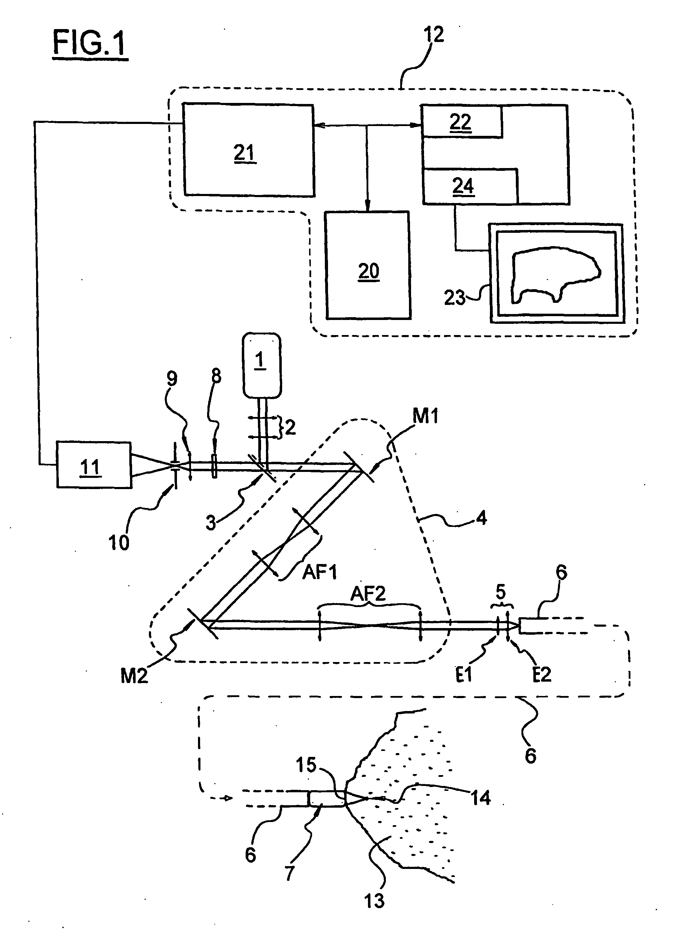

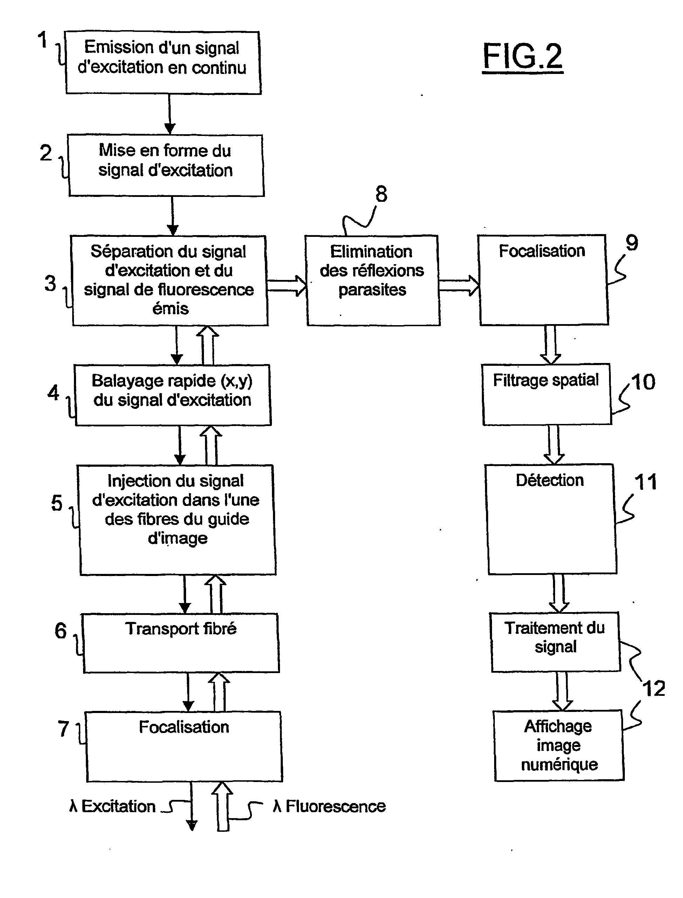

Selon l'exemple choisi et représenté sur les figures 1 et 2, l'appareillage comporte :
- une source lumineuse 1 ;
- des moyens 2 de mise en forme du faisceau d'excitation ;
- des moyens 3 de séparation de longueurs d'onde ;
- des moyens 4 de balayage ;
- des moyens 5 d'injection de faisceau ;
- un guide d'image 6 constitué de fibres optiques souples ;
- une tête optique de focalisation 7 ;
- des moyens 8 de réjection du faisceau d'excitation ;
- des moyens 9 de focalisation du signal de fluorescence ;
- des moyens 10 de filtrage spatial du signal de fluorescence ;
- des moyens 11 de détection du signal de fluorescence ; et
- des moyens 12 de traitement électronique et informatique du signal de fluorescence détecté et de visualisation.

Ces différents éléments sont détaillés ci-après.

La source lumineuse 1 est un laser émettant à une longueur d'onde d'excitation permettant d'exciter une grande gamme de fluorophores, par exemple 488 nm. Pour optimiser l'injection dans l'une des fibres du guide d'image 6, le faisceau d'excitation est circulaire pour pouvoir injecter une fibre de section également circulaire et, pour optimiser le taux d'injection, le laser est de préférence monomode longitudinal pour présenter le meilleur front d'onde possible pour l'injection dans une fibre optique faiblement multimode. Le laser émet de manière continue, et de manière stable (bruit le plus faible possible, <1%). La puissance en sortie disponible est de l'ordre de 20mW. A titre d'exemples, on peut utiliser un laser à puits quantiques (VCSEL), un laser solide pompé par diode, une diode laser ou encore un laser à gaz tel que l'Argon.

En sortie de la source 1, sont placés les moyens 2 de mise en forme du faisceau laser d'excitation. Ils sont constitués d'un système optique afocal de grandissement différent de 1, composé de deux lentilles L1 et L2 qui permettent de modifier le diamètre du faisceau laser. Le grandissement est calculé de sorte que le diamètre du faisceau soit adapté aux moyens d'injection 5 dans une fibre.

Le faisceau laser d'excitation remis en forme est ensuite dirigé vers les moyens 3 prévus pour séparer les longueurs d'ondes d'excitation et de fluorescence. Il s'agit par exemple d'un filtre dichroïque ayant une efficacité de transmission de 98 à 99 % de la longueur d'onde d'excitation et qui réfléchit donc sensiblement les autres longueurs d'onde. Le signal de fluorescence, empruntant au retour le même chemin optique que le signal d'excitation (caractère confocal), sera ainsi envoyé pratiquement totalement vers la voie de détection (8-11). Les moyens de réjection 8 placés sur la voie de détection servent à éliminer totalement les 1 à 2 % de réflexions parasites à la longueur d'onde d'excitation 488 nm qui passent vers la voie de détection (par exemple un filtre de réjection à 488 nm, un filtre passe bande ne permettant par exemple qu'une transmission entre 500 et 600nm, ou un filtre passe-haut qui permet une transmission au dessus de 500 nm).

Les moyens de balayage 4 reprennent ensuite le faisceau d'excitation. Selon l'exemple choisi et représenté sur la figure 1, ces moyens comprennent un miroir M1 résonant à 4 KHz servant à dévier le faisceau horizontalement et donc à réaliser les lignes de l'image, d'un miroir M2 galvanométrique à 15 Hz servant à dévier le faisceau verticalement et donc à réaliser la trame de l'image ; et de deux systèmes afocaux de grandissement unitaire, AF1 situé entre les deux miroirs et AF2 situé après le miroir M2, ces systèmes afocaux étant utilisés pour conjuguer les plans de rotation des deux miroirs M1 et M2 avec le plan d'injection dans l'une des fibres. Selon l'invention, la vitesse de balayage est déterminée pour permettre une observation des tissus in vivo in situ en temps réel. Pour cela, le balayage doit être suffisamment rapide pour qu'il y ait au moins 12 images / s affichées à l'écran pour un mode d'affichage de 640 x 640 pixels correspondant au mode le plus lent. Pour les modes d'affichage ayant moins de pixels, le nombre d'images acquises par seconde sera ainsi toujours supérieur à 12 images / s. En variante, les moyens de balayage peuvent comprendre notamment un miroir rotatif, des composants intégrés de type MEMs (miroirs de balayage X et Y), ou un système acousto-optique.

Le faisceau d'excitation dévié en sortie des moyens de balayage est dirigé vers les moyens optiques 5 afin d'être injecté dans l'une des fibres du guide d'image 6. Ces moyens 5 sont constitués ici de deux ensembles optiques E1 et E2. Le premier ensemble optique E1 permet de corriger en partie les aberrations optiques en bord de champ des moyens de balayage 4, l'injection étant ainsi optimisée sur l'ensemble du champ optique (au centre comme au bord). Le second ensemble optique E2 est destiné à réaliser l'injection proprement dite. Sa focale et son ouverture numérique ont été choisies pour optimiser le taux d'injection dans les fibres optiques du guide 6. Selon un mode de réalisation permettant d'obtenir le critère d'achromaticité, le premier ensemble E1 est constitué d'un doublet de lentilles achromatiques, et le second ensemble E2 de deux doublets de lentilles achromatiques suivi d'une lentille située près du guide d'image. En variante, cette optique d'injection pourrait être constituée de tout autre type d'optiques standards, comme par exemple deux triplets, ou de lentilles à gradient d'indice ou bien d'un objectif de microscope (toutefois plus coûteux).

Le guide d'image 6 est constitué d'un très grand nombre de fibres optiques souples (quelques dizaines de milliers), par exemple 30 000 fibres de 2 µm de diamètre et espacées de 3,3 µm. En pratique, on peut utiliser soit l'ensemble des fibres du guide d'image, soit un sous-ensemble choisi de ces fibres, par exemple centré.

En sortie de la fibre optique, le faisceau laser d'excitation est focalisé par la tête optique 7 dans l'échantillon 13 en un point 14 situé à une profondeur donnée située entre quelques dizaines de µm et une centaine de µm, par rapport à la surface 15 de l'échantillon au contact de laquelle est destinée à être placée la tête optique. Cette profondeur peut être par exemple de 40 µm. La tête optique permet donc de focaliser le flux sortant du guide d'image dans l'échantillon, mais également de collecter le flux de fluorescence revenant de l'échantillon. La tête optique possède un grandissement de 2,4 et une ouverture numérique sur l'échantillon de 0,5. Ces deux paramètres sont choisis afin que le signal de retour se fasse uniquement dans la fibre optique ayant transmis le signal d'excitation et non pas dans des fibres adjacentes et conserver ainsi le filtrage confocal à l'aide d'une fibre. Avec ces valeurs de grandissement et d'ouverture numérique, la résolution axiale est de l'ordre de 10 µm et la résolution latérale de l'ordre de 1,4 µm. L'ouverture numérique est également choisie de manière à optimiser le nombre de photons récupérés qui doit être le plus important possible. La tête optique peut être constituée d'optiques classiques (doublet, triplet, asphérique) et/ou de lentilles à gradient d'indice (GRIN) et/ou de lentilles diffractives, présentant une qualité optique et un chromatisme adaptés à la confocalité, c'est à dire minimisant les aberrations optiques, qui entraîneraient sinon notamment des dégradations sur la profondeur de champ et par conséquent sur la résolution axiale de l'appareillage. En fonctionnement, la tête optique est destinée à être posée au contact de l'échantillon 13. Ce dernier est un tissu biologique ou une culture cellulaire. L'expression de la fluorescence est réalisée soit par un fluorophore que l'on injecte (fluorescence systémique), soit par un fluorophore fabriqué par la cellule elle même par modification d'un gêne (fluorescence transgénique). Dans ces deux cas, le fluorophore re-émet des photons sur une bande spectrale plus ou moins grande pouvant aller d'une dizaine de nanomètres à plus d'une centaine de nanomètres.

Sur la voie de détection, le signal de fluorescence, en sortie du filtre de réjection 8, est ensuite focalisé par les moyens 9, constitués par exemple d'une lentille de détection, dans un trou de filtrage des moyens 10 de filtrage spatial. La focale de la lentille de détection est calculée pour que le signal de fluorescence provenant d'une fibre soit de la taille ou légèrement inférieure à celle du trou de filtrage. Ce dernier permet de conserver la lumière de fluorescence ne provenant que de la fibre illuminée par le faisceau incident. Il permet de rejeter la lumière qui aurait pu être couplée dans les fibres adjacentes à celle qui est illuminée. La taille du trou est calculée pour que l'image d'une fibre s'y inscrive parfaitement. Ici, elle est de 20 µm. Par ailleurs, toujours dans un souci d'optimiser la quantité de photons traversant le trou de filtrage, et donc le flux détecté, les moyens de balayage 4, les moyens d'injection 5, les moyens de focalisation 7 de la tête optique, et les moyens de détection 8, 9 et 10 sont adaptés au fluorophore détecté : ces moyens sont choisis pour être suffisamment achromatiques pour collecter des photons sur la bande la plus large d'émission du fluorophore.

Les moyens de détection 11 ont une sensibilité maximale aux longueurs d'onde de fluorescence étudiées. On peut utiliser par exemple une photodiode à avalanches (APD) ou bien un photomultiplicateur. Par ailleurs, selon l'invention, la bande passante est choisie pour optimiser le temps d'intégration du signal de fluorescence. Elle est de 1,5 MHz, ce qui correspond à la fréquence d'échantillonnage minimale du guide d'image avec un temps d'intégration optimisé sur chaque pixel.

Les moyens électroniques et informatiques 12 de commande, d'analyse et de traitement numérique du signal détecté et de visualisation comprennent les cartes suivantes:
- une carte de synchronisation 20 qui a pour fonctions :
   - de commander de manière synchronisée le balayage, c'est-à-dire le mouvement des miroirs ligne M1 et trame M2 ;
   - de connaître à tout instant la position du spot laser ainsi balayé ;
      et
   - de gérer toutes les autres cartes par l'intermédiaire d'un micro-contrôleur lui-même pouvant être piloté ;
- une carte détecteur 21 qui comprend un circuit analogique qui réalise notamment une adaptation d'impédance, un convertisseur analogique numérique puis un composant logique programmable (par exemple un circuit FPGA) qui met en forme le signal ;
- une carte d'acquisition numérique 22 qui permet de traiter un flot de données numériques à fréquence variable et de l'afficher sur un écran 23 ;
- une carte graphique 24.

En variante, on peut utiliser une seule carte regroupant les fonctionnalités de ces différentes cartes.

Le traitement d'image se fait de la manière suivante.

A la mise en place d'un guide d'image dans l'appareillage, une première opération est effectuée pour reconnaître le motif des fibres dans le guide d'image, et donc connaître l'emplacement réel de chaque fibre destinée à être utilisée.

Les opérations suivantes sont également réalisées préalablement à l'utilisation de l'appareillage :
- la détermination du taux d'injection propre à chaque fibre, à l'aide d'un échantillon homogène, ce taux d'injection pouvant varier d'une fibre à une autre ; et
- la mesure de l'image de fond, effectuée sans échantillon.

Ces deux opérations peuvent être réalisées régulièrement en fonction de la fréquence d'utilisation de l'appareillage. Les résultats obtenus vont être utilisés pour corriger en fonctionnement le signal numérique en sortie de la carte détecteur.

En fonctionnement, selon l'invention 2 groupes de traitements sont effectués sur le signal numérique en sortie de la carte détecteur :
Le premier groupe consiste dans un premier temps à corriger le signal numérique notamment pour tenir compte du taux propre réel d'injection de la fibre dont est issu ledit signal et pour lui soustraire la partie du flux correspondant à l'image de fond. Cela permet de ne traiter qu'un signal correspondant réellement à l'échantillon observé. On utilise pour ce groupe de traitement un algorithme de calcul classique qui est optimisable pour respecter la contrainte du temps réel.
Le second groupe consiste ensuite, à partir du signal corrigé, à reconstruire l'image numérique qui sera visualisée par le praticien. Le but du traitement effectué est d'offrir à la visualisation une image numérique reconstituée qui ne soit pas simplement une mosaïque d'éléments d'image correspondant chacun à un signal numérique corrigé d'une fibre mis côte à côte, mais d'offrir une image numérique reconstituée qui ne fasse plus apparaître les fibres. On utilise pour cela un algorithme destiné à effectuer un certain nombre d'opérations sur chaque pixel, l'algorithme étant choisi pour respecter la contrainte de temps réel, c'est à dire qu'il doit représenter un bon compromis entre la complexité des opérations demandées, la qualité du résultat que l'on peut obtenir et le temps de calcul. A titre d'exemple, on peut utiliser un algorithme de filtrage passe-bas Gaussien.

Le fonctionnement de l'appareillage est le suivant. La source 1 produit un faisceau parallèle circulaire d'excitation à une longueur d'onde de 488 nm, qui est ensuite remis en forme dans le système afocal 2 afin de lui donner la taille adéquate pour la meilleure injection possible dans le coeur d'une fibre. Ce faisceau est ensuite envoyé vers le système de séparation dichroïque 3 qui réfléchit la longueur d'onde d'excitation. Le faisceau incident est ensuite dévié angulairement dans le temps dans les deux directions de l'espace par le système de balayage optomécanique de miroirs 4, et injecté grâce aux moyens optiques d'injection 5 dans l'une des fibres du guide d'image 6. Les moyens électroniques 12 servent à commander l'injection à un instant donné de l'une des fibres optiques du guide d'image en déviant angulairement le faisceau au moyen des miroirs, et ce point par point pour une ligne donnée, et ligne après ligne, pour constituer l'image. En sortie du guide, la lumière émergeant de la fibre injectée est focalisée dans l'échantillon grâce à la tête optique 7 en un point situé à une profondeur donnée située sensiblement entre quelques dizaines de µm et une centaine de µm. Grâce au balayage, l'échantillon est illuminé point par point. A chaque instant, le spot illuminant le tissu émet alors un signal de fluorescence qui a la particularité d'être décalé vers des plus grandes longueurs d'onde. Ce signal de fluorescence est capté par la tête optique 7, puis suit le chemin inverse du faisceau d'excitation jusqu'au filtre dichroïque 3 qui va transmettre le signal de fluorescence vers la voie de détection. Les réflexions parasites se produisant à la longueur d'onde d'excitation vont ensuite être rejetées par le filtre de réjection 8. Enfin, le signal de fluorescence est focalisé dans le trou de filtrage 10 pour ne sélectionner que la lumière provenant de la fibre excitée et les photons sont détectés par la photodiode à avalanche 11. Le signal détecté est ensuite numérisé et corrigé. Les signaux détectés, les uns après les autres, sont traités en temps réel grâce au traitement d'image décrit plus haut pour permettre la reconstruction d'une image en temps réel visualisée à l'écran.

Le guide d'image 6 utilisé pour cet appareillage peut être composé sensiblement de 5 000 à 100 000 fibres optiques souples, selon le diamètre extérieur du guide que l'on souhaite, qui est lui même fonction de l'application visée (endoscopique, taille de champ recherché, etc.). Il peut s'agir par exemple de guides d'images commercialisés par la société FUJIKURA. Le diamètre de coeur des fibres est de préférence compris entre 1 et 4 µm. Cela conduit à une divergence de faisceau en sortie de la fibre présentant un angle d'environ 18° pour une ouverture numérique de 0,42 dans l'air. Un diamètre de coeur de 1µm peut être obtenu grâce à un procédé d'étirement de l'extrémité de l'ensemble du guide d'image. L'extrémité du guide 6 est polie et ne comporte pas de moyens optiques. Le polissage a pour but de conférer le même état de surface à la face du guide et donc aux fibres nues au contact du tissu pour, d'une part que le fond de l'image obtenu soit le plus homogène possible et, d'autre part, pour supprimer les éventuels problèmes d'adhérence des fibres avec le tissu qui risquerait de l'abîmer. Le polissage peut être plan ou arrondi pour épouser la forme du tissu. L'ouverture numérique de chaque fibre optique est de préférence choisie la plus grande possible pour collecter le maximum de photons de fluorescence, c'est à dire par exemple de 0,42. La distance inter-coeur confère la résolution latérale de l'image obtenue (distance entre deux points de l'image). Plus cette distance sera petite, meilleure sera la résolution, en revanche cela se fera au détriment de la taille du champ à imager. Il convient donc de trouver un bon compromis entre le nombre de fibres du guide et la distance inter-coeur entre les fibres pour obtenir une bonne résolution latérale (entre 2 et 8 µm) avec une taille de champ appropriée pour observer les éléments tissulaires souhaités.

Deux exemples de guides pouvant convenir selon l'invention sont donnés ci-après :

| | Exemple 1 | Exemple 2 |
|---|---|---|
| Nombre de fibres | 6000 | 30 000 |
| Diamètre de champ imagé | 300 µm | 650 µm |
| Diamètre extérieur du guide | 330 µm | 750 µm |
| Diamètre de coeur d'une fibre | 3 µm | 1,9 µm |
| Distance inter-coeur | 4 µm | 3,3 µm |
| Profondeur maximale d'intégration du signal depuis la surface (schématisé par le plan P sur la figure 1) ou résolution axiale | 15-20 µm | 10-15 µm |
| Résolution latérale | 4 µm | 3,3 µm |

## Revendications

1. Procédé pour réaliser une image confocale de fluorescence in vivo in situ, le procédé utilisant un guide d'image fait de plusieurs milliers de fibres optiques et consistant à balayer point par point un tissu dans un plan subsurfacique, chaque point correspondant à un signal d'excitation émis par une source continue, dévié et injecté dans l'une des fibres optiques dudit faisceau puis focalisé à la sortie de ladite fibre dans ledit plan, chaque point émettant en retour un signal de fluorescence collecté par ladite fibre optique, puis détecté et numérisé pour former un élément d'image, le procédé comportant les étapes suivantes :
l'on dévie le signal d'excitation à une vitesse correspondant à l'acquisition d'un minimum de 12 images par seconde dans le cas d'un mode d'affichage maximal de 640×640 pixels,
- l'on détecte le signal de fluorescence à une fréquence de détection supérieure ou égale à une fréquence minimale d'échantillonnage des fibres une à une qui est sensiblement égale à 1,5MHz pour ledit minimum de 12 images par seconde dans le cas d'un mode d'affichage de 640x640 pixels.

2. Procédé selon la revendication 1, **caractérisé par** une ouverture numérique de l'optique de focalisation comprise sensiblement entre 0,5 et 1.

3. Procédé selon l'une des revendications précédentes, **caractérisé en ce que** l'on règle une vitesse de déviation du faisceau d'excitation en déterminant une fréquence de résonance rapide d'un miroir résonnant ligne et une fréquence de résonance lente d'un miroir galvanométrique trame.

4. Procédé selon l'une des revendications précédentes, **caractérisé en ce que** l'on utilise des moyens optiques de déviation, d'injection, de focalisation et de détection présentant un degré d'achromaticité permettant de collecter des photons sur toute la largeur de la bande d'émission du fluorophore excité.

5. Procédé selon l'une des revendications précédentes, **caractérisé par** une efficacité quantique de la détection aux longueurs d'ondes de fluorescence à détecter d'au moins 50%.

6. Procédé selon l'une des revendications précédentes, **caractérisé par** une étape préalable de détection de l'emplacement des fibres du guide d'image destinées à être utilisées.

7. Procédé selon l'une des revendications précédentes, **caractérisé par** une étape préalable de détermination du taux réel d'injection propre à chaque fibre.

8. Procédé selon l'une des revendications précédentes, **caractérisé par** une étape préalable de détermination du flux collecté correspondant à l'image de fond.

9. Procédé selon les revendications 7 et 8, **caractérisé par** une étape de correction du signal numérisé en provenance d'une fibre par soustraction du flux correspondant à l'image de fond et adaptation au taux réel d'injection propre de ladite fibre.

10. Procédé selon la revendication 9, **caractérisé par** une étape de reconstruction de l'image à partir du signal corrigé.

11. Procédé selon la revendication 10, **caractérisé en ce que** l'étape de reconstruction d'image comprend un filtrage passe- bas Gaussien.

12. Procédé selon l'une quelconque des revendications précédentes, **caractérisé en ce que** le signal d'excitation est adapté à un fluorophore de type endogène du tissu cellulaire.

13. Appareillage d'imagerie de fluorescence confocale fibrée in vivo in situ pour la mise en oeuvre du procédé selon l'une des revendications précédentes, comprenant :
- une guide d'image (6) comprenant plusieurs milliers de fibres optiques;
- une source (1) émettant en continu à la longueur d'onde d'excitation d'au moins un fluorophore ciblé,
- des moyens de balayage (4) et d'injection (5) fibre à fibre dans le temps du faisceau d'excitation produit par la source (1) en lignes et en colonnes dans un plan XY correspondant à la section d'entrée du guide d'image (6);
- des moyens (3) de séparation de la longueur d'onde d'excitation et des longueurs d'onde de fluorescence ;
- des moyens de détection (11) du signal de fluorescence ; et
- des moyens (12) de traitement du signal détecté permettant la réalisation d'une image ;
à l'extrémité distale étant ménagée une tête optique (7), destinée à être mise en contact avec le tissu observé (13), permettant de focaliser le faisceau d'excitation,
les moyens de balayage étant adaptés à déplacer le faisceau d'excitation à une vitesse correspondant à l'obtention d'un minimum de 12 images par seconde dans le cas d'un mode d'affichage maximal de 640x640 pixels; et
les moyens de détection présentant une bande passante dont la fréquence est supérieure ou égale à une fréquence minimale d'échantillonnage des fibres une à une qui est égale à 1,5MHz pour ledit minimum de 12 images par seconde dans le cas d'un mode d'affichage de 640x640 pixels.

14. Appareillage selon la revendication 13, **caractérisé en ce que** le faisceau d'excitation produit par la source (1) est monomode longitudinal présentant une qualité de front d'onde optimale pour l'injection dans une fibre optique faiblement multimode.

15. Appareillage selon la revendication 13 ou 14, **caractérisé en ce que** la section d'une fibre étant circulaire, le faisceau d'excitation produit par la source est circulaire de manière à optimiser l'injection dans une fibre.

16. Appareillage selon l'une des revendications 13 à 15, **caractérisé par** des moyens (2) de mise en forme du faisceau utilisés en sortie de la source (1) pour mettre en forme le faisceau d'excitation afin de l'adapter aux moyens d'injection (5) dans le guide d'image (6).

17. Appareillage selon l'une des revendications 13 à 16, **caractérisé en ce que** les moyens pour séparer les longueurs d'onde d'excitation et de fluorescence comprennent un filtre dichroïque (3) ayant un maximum d'efficacité à la longueur d'onde d'excitation.

18. Appareillage selon l'une des revendications 13 à 17, **caractérisé par** des moyens de réjection (8) placé en amont des moyens de détection (11) et adaptés à éliminer la longueur d'onde d'excitation.

19. Appareillage selon l'une des revendications 13 à 18, **caractérisé en ce que** les moyens de balayage (4) comprennent un miroir ligne résonnant (M1), un miroir galvanométrique trame (M2), un premier système optique afocal de grandissement unitaire (AF1) permettant de conjuguer les deux miroirs et un second système afocal (AF2) de grandissement unitaire permettant de conjuguer les plans de rotation des deux miroirs avec le plan d'injection dans l'une des fibres.

20. Appareillage selon l'une des revendications 13 à 19, **caractérisé en ce que** les moyens optiques de la tête optique (7), les moyens de balayage (4), les moyens d'injection 5) et les moyens de détection présentent un degré d'achromatisation permettant de collecter des photons sur toute la largeur de la bande spectrale du signal de fluorescence.

21. Appareillage selon l'une des revendications 13 à 20, **caractérisé en ce que** les moyens d'injection (5) comprennent deux ensembles optiques (E1,E2), le premier ensemble (E1) étant adapté à corriger les aberrations optiques en bordure de champ des moyens de balayage (4) et le second ensemble (E2) étant adapté à réaliser l'injection proprement dite dans l'une des fibres du guide d'image (6).

22. Appareillage selon la revendication 21, **caractérisé en ce que** le premier ensemble optique (E1) comprend un doublet et le second ensemble optique (E2) comprend deux doublets suivi d'une lentille.

23. Appareillage selon l'une des revendications 13 à 22, **caractérisé par** un trou de filtrage (10) placé avant les moyens de détection (11) dont le diamètre est choisi pour que l'image d'une fibre s'y inscrive.

24. Appareillage selon la revendication 23, **caractérisé par** des moyens de focalisation (9) du signal de fluorescence sur le trou de filtrage (10).

25. Appareillage selon l'une des revendications 13 à 24, **caractérisé en ce que** la source est adaptée à exciter un fluorophore endogène présent dans le tissu cellulaire.

## Claims

1. Method for the realization of a confocal fluorescence in vivo in situ image, the method using an image guide made of several thousands of optical fibres and consisting of the point by point scanning of a tissue in a subsurface plane, each point corresponding to an excitation signal emitted by a continuous source, deflected and injected into one of the optical fibres of said bundle then focussed on the exit of said fibre in said plane, each point emitting in return a fluorescence signal collected by said optical fibre, then detected and digitized to form an image element, the method comprising following steps:
- the excitation signal is deflected at a speed corresponding to acquisition of at least 12 images per second in the case of a maximum display mode of 640x640 pixels,
- the fluorescence signal is detected at a detection frequency greater than or equal to a minimum sampling frequency of the fibres one-by-one which is substantially equal to 1.5MHz for said at least 12 images per second in the case of a maximum display mode of 640x640 pixels.

2. Method according to claim 1, **characterized by** a numerical aperture of the focussing optics between approximately 0.5 and 1.

3. Method according to one of the preceding claims, **characterized in that** the deflection speed of the excitation beam is adjusted by determining a rapid-resonance frequency of a resonating line mirror and a slow-resonance frequency of a galvanometric frame mirror.

4. Method according to one of the preceding claims, **characterized in that** optical deflection, injection, focussing and detection means are used having a degree of achromaticity which allows the collection of photons over the whole of the emission band of the excited fluorophore.

5. Method according to one of the preceding claims, **characterized by** a quantum efficiency of detection at the fluorescence wavelengths to be detected of at least 50 %.

6. Method according to one of the preceding claims, **characterized by** a prior step of detecting the placement of the fibres of the image guide which are intended to be used.

7. Method according to one of the preceding claims, **characterized by** a prior step of determining the real injection rate particular to each fibre.

8. Method according to one of the preceding claims, **characterized by** a prior step of determining the collected flux corresponding to the background image.

9. Method according to claims 7 and 8, **characterized by** a step of correcting the digitized signal coming from a fibre by subtraction of the flux corresponding to the background image and adaptation to the real rate of injection which is particular to said fibre.

10. Method according to claim 9, **characterized by** a step of reconstructing the image from the corrected signal.

11. Method according to claim 10, **characterized in that** the step of reconstructing the image comprises a Gaussian low-pass filtering.

12. Method according to one of the preceding claims, **characterized in that** the excitation signal is adapted to an endogenous type of fluorophore of the cell tissue.

13. Apparatus for in situ in vivo fibred optic confocal fluorescence imaging for the implementation of the method according to one of preceding claims, comprising:
- an image guide (6) comprising several thousands of optical fibres;
- a source (1) emitting continuously at the excitation wavelength of at least one targeted fluorophore,
- means for scanning (4) and injection (5) fibre by fibre over time of the excitation beam produced by the source (1) by lines and by columns in a XY plane corresponding to the entry section of the image guide (6);
- means (3) for separating the excitation wavelength and the fluorescence wavelengths;
- means for detection (11) of the fluorescence signal; and
- means (12) for processing the detected signal allowing the realization of an image;
an optical head (7) being arranged at the distal end, intended to be brought into contact with the observed tissue (13), allowing the excitation beam to be focussed;
the scanning means being suitable for moving the excitation beam at a speed corresponding to the obtaining of at least 12 images per second in the case of a maximum display mode of 640x640 pixels; and
the detection means having a pass-band whose frequency is greater than or equal to a minimum sampling frequency of the fibres one-by-one which is substantially equal to 1.5MHz for said at least 12 images per second in the case of a maximum display mode of 640x640 pixels.

14. Apparatus according to claim 13, **characterized in that** the excitation beam produced by the source (1) is of the longitudinal monomode type presenting an optimum wave front quality for the injection into a slightly multimode optical fibre.

15. Apparatus according to claim 13 or 14, **characterized in that**, the section of a fibre being circular, the excitation beam produced by the source is circular so as to optimize the injection into a fibre.

16. Apparatus according to one of claims 13 to 15, **characterized by** means (2) for shaping the beam used on the exit of the source (1) in order to shape the excitation beam so as to adapt it to the injection means (5) in the image guide (6).

17. Apparatus according to one of claims 13 to 16, **characterized in that** the means for separating the excitation and fluorescence wavelengths comprise a dichroic filter (3) having a maximum efficiency at the excitation wavelength.

18. Apparatus according to one of claims 13 to 17, **characterized by** rejection means (8) placed upstream of the detection means (11) and suitable for eliminating the excitation wavelength.

19. Apparatus according to one of claims 13 to 18, **characterized in that** the scanning means (4) comprise a resonating line mirror (M1), a galvanometric frame mirror (M2), a first afocal optical system (AF1) with unitary magnification adapted for the conjugation of the two mirrors and a second afocal system (AF2) with unitary magnification adapted for the conjugation of the rotation planes of the two mirrors with the injection plane in one of the fibres.

20. Apparatus according to one of claims 13 to 19, **characterized in that** the optical means of the optical head (7), the scanning means (4), the injection means (5) and the detection means present a degree of achromaticity adapted for the collection of the photons over the whole of the width of the spectral band of the fluorescence signal.

21. Apparatus according to one of claims 13 to 20, **characterized in that** the injection means (5) comprise two optical units (E1, E2), the first unit (E1) being adapted for correcting the optical aberrations at the edge of the field of the scanning means (4) and the second unit (E2) being adapted for carrying out the actual injection in one of the fibres of the image guide (6).

22. Apparatus according to claim 21, **characterized in that** the first optical unit (E1) comprises a doublet and the second optical unit (E2) comprises two doublets followed by a lens.

23. Apparatus according to one of claims 13 to 22, **characterized by** a filtering hole (10) placed in front of the detection means (11) whose diameter is chosen so that the image of a fibre fits into it.

24. Apparatus according to claim 23, **characterized by** means (9) for focussing the fluorescence signal on the filtering hole (10).

25. Apparatus according to one of claims 13 to 24, **characterized in that** the source is adapted to excite an endogenous fluorophore which is present in the cell tissue.

## Patentansprüche

1. Verfahren zur Herstellung eines konfokalen Fluoreszenzbildes in vivo und in situ, wobei das Verfahren einen Bildleiter benutzt, der aus mehreren Tausenden von optischen Fasern gebildet ist, und darin besteht, dass ein Gewebe Punkt für Punkt in einer Ebene unter der Oberfläche abgetastet wird, wobei jeder Punkt einem von einer kontinuierlichen Quelle gesendeten Anregungssignal entspricht, das abgelenkt und in eine der optischen Fasern des Bündels eingeleitet wird und dann am Austritt aus der Faser auf diese Ebene fokussiert wird, wobei jeder Punkt ein Fluoreszenzsignal zurücksendet, das von der optischen Faser gesammelt wird, dann erfasst und digitalisiert wird, um ein Bildelement zu bilden, wobei das Verfahren die folgenden Schritte umfasst:
- man lenkt das Anregungssignal mit einer Geschwindigkeit ab, die der Erfassung eines Minimums von 12 von Bildern pro Sekunde im Fall eines Modus der maximalen Anzeige von 640x640 Pixel entspricht,
- man erfasst das Fluoreszenzsignal mit einer Erfassungsfrequenz von größer als oder gleich einer Mindestfrequenz der Abtastung der Fasern einer nach der anderen, die im Wesentlichen gleich 1,5 MHz bei dem Minimum von 12 Bildern pro Sekunde im Fall eines Anzeigemodus von 640x640 Pixel ist.

2. Verfahren nach Anspruch 1, **gekennzeichnet durch** eine numerische Apertur der Fokussierungsoptik im Wesentlichen zwischen 0,5 und 1.

3. Verfahren nach einem der vorhergehenden Ansprüche, **dadurch gekennzeichnet, dass** man eine Geschwindigkeit der Ablenkung des Anregungsstrahls einstellt, indem man eine schnelle Resonanzfrequenz eines Zeilen-Resonanzspiegels und eine langsame Resonanzfrequenz eines galvanometrischen Spalten-Spiegels bestimmt.

4. Verfahren nach einem der vorhergehenden Ansprüche, **dadurch gekennzeichnet, dass** man optische Ablenkungs-, Einleitungs-, Fokussierungs- und Erfassungsmittel verwendet, die einen Achromatizitätsgrad aufweisen, der es gestattet, Photonen auf der ganzen Breite des Emissionsbandes des angeregten Fluorophors zu sammeln.

5. Verfahren nach einem der vorhergehenden Ansprüche, **gekennzeichnet durch** eine Quantenwirksamkeit der Erfassung bei den zu erfassenden Fluoreszenzwellenlängen von mindestens 50 %.

6. Verfahren nach einem der vorhergehenden Ansprüche, **gekennzeichnet, durch** einen vorhergehenden Schritt der Erfassung der Lage der Fasern des Bildleiters, die dazu bestimmt sind, verwendet zu werden.

7. Verfahren nach einem der vorhergehenden Ansprüche, **gekennzeichnet durch** einen vorhergehenden Schritt der Bestimmung des tatsächlichen Einleitungsgrads jeder Faser.

8. Verfahren nach einem der vorhergehenden Ansprüche, **gekennzeichnet durch** einen vorhergehenden Schritt der Bestimmung des dem Hintergrundbild entsprechenden gesammelten Flusses.

9. Verfahren nach den Ansprüche 7 und 8, **gekennzeichnet durch** einen Schritt der Korrektur des von einer Faser kommenden digitalisierten Signals **durch** Subtraktion des dem Hintergrundbild entsprechenden Flusses und Anpassung an den tatsächlichen Einleitungsgrad dieser Faser.

10. Verfahren nach Anspruch 9, **gekennzeichnet durch** einen Schritt der Rekonstruktion des Bilds aus dem korrigierten Signal.

11. Verfahren nach Anspruch 10, **dadurch gekennzeichnet, dass** der Bildrekonstruktionsschritt eine Gauß'sche Tiefpassfilterung umfasst.

12. Verfahren nach einem der vorhergehenden Ansprüche, **dadurch gekennzeichnet, dass** das Anregungssignal an ein endogenes Fluorophor des Zellgewebes angepasst ist.

13. Vorrichtung zur konfokalen Fluoreszenzbildgebung mit optischen Fasern in vivo und in situ für die Durchführung des Verfahrens nach einem der vorhergehenden Ansprüche, umfassend:
- einen Bildleiter (6), umfassend mehrere Tausende von optischen Fasern;
- eine Quelle (1), die kontinuierlich mit der Anregungswellenlänge mindestens ein Ziel-Fluorophor sendet,
- Mittel zur Abtastung (4) und Einleitung (5) Faser für Faser in der Zeit des durch die Quelle (1) erzeugten Anregungsbündels in Zeilen und in Spalten in einer dem Eintrittsquerschnitt der Bildführung (6) entsprechenden Ebene XY;
- Mittel (3) zur Trennung der Anregungswellenlänge und der Fluoreszenzwellenlängen;
- Mittel (11) zur Erfassung des Fluoreszenzsignals; und
- Mittel (12) zur Verarbeitung des erfassten Signals, die die Herstellung eines Bilds gestatten;
wobei am distalen Ende ein optischer Kopf (7) vorgesehen ist, der dazu bestimmt ist, mit dem beobachteten Gewebe (13) in Kontakt gebracht zu werden, und die Fokussierung des Anregungsbündels gestattet,
wobei die Abtastmittel dafür ausgelegt sind, das Anregungsbündel mit einer Geschwindigkeit zu bewegen, die dem Erhalten eines Minimums von 12 Bildern pro Sekunde im Fall eines Modus der maximalen Anzeige von 640x640 Pixel entspricht; und
die Erfassungsmittel einen Durchlassbereich aufweisen, dessen Frequenz höher als oder gleich einer Mindestfrequenz der Abtastung der Fasern einer nach der anderen ist, die im Wesentlichen gleich 1,5 MHz bei diesem Minimum von 12 Bildern pro Sekunde im Fall eines Anzeigemodus von 640x640 Pixel ist.

14. Vorrichtung nach Anspruch 13, **dadurch gekennzeichnet, dass** das durch die Quelle (1) erzeugte Anregungsbündel longitudinal monomodal mit einer optimalen Wellenfrontqualität für die Einleitung in eine schwach multimodale optische Faser ist.

15. Vorrichtung nach Anspruch 13 oder 14, **dadurch gekennzeichnet, dass**, wenn der Querschnitt einer Faser kreisförmig ist, das durch die Quelle erzeugte Anregungsbündel kreisförmig ist, so dass die Einleitung in eine Faser optimiert wird.

16. Vorrichtung nach einem der Ansprüche 13 bis 15, **gekennzeichnet durch** Mittel (2) zur Formung des Bündels, die am Austritt der Quelle (1) verwendet werden, um das Anregungsbündel in Form zu bringen, um es an die Mittel (5) zur Einleitung in den Bildleiter (6) anzupassen.

17. Vorrichtung nach einem der Ansprüche 13 bis 16, **dadurch gekennzeichnet, dass** die Mittel zum Trennen der Anregungs-und Fluoreszenzwellenlängen einen dichroitischen Filter (3) mit einem Wirksamkeitsmaximum bei der Anregungswellenlänge umfassen.

18. Vorrichtung nach einem der Ansprüche 13 bis 17, **gekennzeichnet durch** Wiedereinleitungsmittel (8), die stromauf der Erfassungsmittel (11) angeordnet sind und dafür ausgelegt sind, die Anregungswellenlänge zu entfernen.

19. Vorrichtung nach einem der Ansprüche 13 bis 18, **dadurch gekennzeichnet, dass** die Abtastmittel (4) einen Zeilen-Resonanzspiegel (M1), einen galvanometrischen Spalten-Spiegel (M2), ein erstes afokales optisches System mit einfacher Vergrößerung (AF1), das es gestattet, die beiden Spiegel zu verbinden, und ein zweites afokales System (AF2) mit einfacher Vergrößerung umfassen, das es gestattet, die Rotationsebenen der beiden Spiegel mit der Ebene der Einleitung in eine der Fasern zu verbinden.

20. Vorrichtung nach einem der Ansprüche 13 bis 19, **dadurch gekennzeichnet, dass** die optischen Mittel des optischen Kopfes (7), die Abtastmittel (4), die Einleitungsmittel (5) und die Erfassungsmittel einen Achromatisierungsgrad aufweisen, der es gestattet, Photonen auf der ganzen Breite des Spektralbands des Fluoreszenzsignals zu sammeln.

21. Vorrichtung nach einem der Ansprüche 13 bis 20, **dadurch gekennzeichnet, dass** die Einleitungsmittel (5) zwei optische Einheiten (E1, E2) umfassen, wobei die erste Einheit (E1) dafür ausgelegt ist, die optischen Aberrationen am Feldrand der Abtastmittel (4) zu korrigieren, und die zweite Einheit (E2) dafür ausgelegt ist, die eigentliche Einleitung in eine der Fasern des Bildleiters (6) durchzuführen.

22. Vorrichtung nach Anspruch 21, **dadurch gekennzeichnet, dass** die erste optische Einheit (E1) ein Dublett umfasst und die zweite optische Einheit (E2) zwei Dubletts umfasst, auf die eine Linse folgt.

23. Vorrichtung nach einem der Ansprüche 13 bis 22, **gekennzeichnet durch** ein vor den Erfassungsmitteln (11) angeordnetes Filterloch (10), dessen Durchmesser so gewählt ist, dass sich das Abbild einer Faser darin einschreibt.

24. Vorrichtung nach Anspruch 23, **gekennzeichnet durch** Mittel (9) zur Fokussierung des Fluoreszenzsignals auf das Filterloch (10).

25. Vorrichtung nach einem der Ansprüche 13 bis 24, **dadurch gekennzeichnet, dass** die Quelle dafür ausgelegt ist, ein im Zellgewebe vorhandenes endogenes Fluorophor anzuregen.
